# FASCICULE DE BREVET EUROPEEN

(11) **EP 3 082 701 B1**
(45) Date de publication et mention de la délivrance du brevet: **30.10.2019**
(21) Numéro de dépôt: 14830692.1
(22) Date de dépôt: 17.12.2014
(51) Int. Cl.: A61F 13/537, A61F 13/02

(54) **ARTICLE POUR ABSORBER UN LIQUIDE PHYSIOLOGIQUE, NOTAMMENT PANSEMENT**
ARTIKEL ZUR ABSORPTION EINER PHYSIOLOGISCHEN FLÜSSIGKEIT, WIE ETWA EIN VERBAND
ARTICLE FOR ABSORBING A PHYSIOLOGICAL LIQUID, SUCH AS A DRESSING

(30) Priorité: 19.12.2013 FR 1362987
(43) Date de publication de la demande: 26.10.2016
(73) Titulaire: Commissariat à l'Énergie Atomique et aux Énergies Alternatives, 75015 Paris (FR); Urgo Recherche Innovation et Développement, 21300 Chenove (FR)
(72) Inventeur: REVOL-CAVALIER, Frédéric, 38180 Seyssins (FR); LAMOISE, Michel, 21110 Bessey Les Citeaux (FR); MESSAOUD, Mouna, Sfax 3000 (TN); PERNOT, Jean-Marc, 21000 Dijon (FR)
(74) Mandataire: Nony
(86) Numéro de dépôt international: PCT/IB2014/067009
(87) Numéro de publication internationale: WO 2015/092702

(56) Documents cités:
- WO-A1-00/42958
- WO-A1-95/17869
- WO-A1-2009/145703
- WO-A1-2014/016759
- DE-U1-202012 101 743
- US-A1- 2008 195 017
- US-A1- 2013 143 020

## Description

La présente invention concerne des articles destinés à venir en contact avec des liquides physiologiques, en particulier des liquides sécrétés par la peau, une plaie et/ou des muqueuses, pour gérer la propagation, le stockage et/ou l'évaporation de ces liquides et, plus particulièrement mais non exclusivement, des pansements à appliquer sur une plaie.

La gestion de la propagation des liquides ainsi que leur stockage sont des problèmes complexes à résoudre pour lesquels les articles proposés dans le domaine des pansements et des produits d'hygiène ne fournissent pas aujourd'hui des solutions entièrement satisfaisantes.

Ces articles doivent en effet satisfaire à un cahier des charges qui comprend des exigences antagonistes.

Une première exigence est d'éloigner le plus loin et le plus rapidement possible les liquides afin d'éviter à cause de leur accumulation tout phénomène de macération ou d'irritation au niveau de la peau, de la plaie ou des muqueuses. Non seulement les liquides ne doivent pas s'accumuler, mais il est aussi préférable d'éviter leur migration latérale à partir du lieu de sécrétion, pour ne pas augmenter l'étendue de la zone humidifiée, à des fins de garantir une meilleure hygiène et contribuer au confort de l'utilisateur.

Ceci est particulièrement important lors de l'utilisation d'un pansement. Dans ce cas, il est primordial d'éviter que la peau située en bordure de la lésion, appelée peau périlésionnelle, qui est très fragile, soit humide, car cela peut provoquer son altération, favorisant par exemple une infection et/ou irritation.

Ainsi, il est souhaitable que le pansement éloigne rapidement les liquides corporels sécrétés par la plaie de leur lieu de sécrétion. Un tel éloignement rapide assure une meilleure hygiène et des conditions de cicatrisation améliorées.

Une seconde exigence est de stocker ces liquides et d'éviter leur retour vers la peau, la plaie ou les muqueuses. Un stockage important permet d'augmenter la durée d'utilisation de l'article. Dans le cas d'un pansement, cette durée d'utilisation est particulièrement importante car elle permet de diminuer le risque d'altérer le processus de cicatrisation de la plaie en changeant ce dernier moins souvent.
Enfin cet éloignement et ce stockage doivent fonctionner en parallèle le plus longtemps possible quelle que soit la capacité de la zone de stockage et en particulier le niveau de liquide absorbé dans la zone de stockage.

Il est donc souhaitable de disposer d'articles qui permettent de gérer la propagation et le stockage des liquides, en particulier des liquides sécrétés par une plaie, la peau ou les muqueuses, en éloignant le plus rapidement possible ces derniers de leur point de sécrétion pour éviter leur accumulation et accroître la durée d'utilisation de l'article.

L'utilisation dans le domaine des pansements de bandes de transfert de liquides pour éloigner ou répartir ces derniers afin de les évaporer et/ou les stocker est connue depuis longtemps.

La demande EP 0541391 propose d'incorporer entre la couche absorbante et le support une couche qui favorise l'étalement des liquides pour optimiser leur évaporation. Toutefois, cette solution est loin d'être optimale, car quand la couche d'étalement est saturée d'eau l'évaporation devient moins effective et surtout les liquides ont tendance à redescendre dans les parties de la couche absorbante non encore en contact avec les liquides. On retrouve alors les risques liés à l'accumulation des liquides au niveau de la plaie et de la peau périlésionnelle, comme par exemple la macération et les possibilités d'infection. On diminue aussi la capacité d'absorption de la couche absorbante, voire on entraine sa déformation, suite à la création de gradients de teneur en liquide en son sein.

La demande WO 2009 145703 A1 divulgue un pansement pouvant transférer les exsudats absorbés par une couche absorbante placée sous la bande de compression vers un réservoir extérieur. Le transfert d'une partie de l'exsudat est réalisé à partir de bandelettes drainantes qui sont des non tissés hydrophiles à base de fibres absorbantes. Toutefois l'optimisation de la vitesse de migration des liquides de telles bandes de transfert et la conservation de cette vitesse de migration au cours du temps quand la bande est totalement humidifiée et / ou le réservoir de stockage se remplit n'ont pas à ce jour été étudiées.

Pour optimiser la gestion des liquides il serait donc souhaitable de disposer d'articles qui comportent des structures de transfert qui présentent une vitesse de migration des liquides optimisée pour drainer ces derniers d'un premier point vers un deuxième point dans le but de leur stockage et/ ou leur évaporation.

Il est également connu la demande de brevet DE 20 2012 101743 divulguant un pansement comportant une couche plane présentant au moins deux ailes connectées entre elles par une portion centrale et arrangées autour de cette dernière. Les ailes sont obtenues par découpes de la couche plane. La forme particulière de la couche plane permet de faciliter l'application du pansement sur des cavités ou des parties du corps ayant une topologie convexe ou irrégulière. Le fait que les ailes soient dans un même matériau que la couche plane limite la rapidité d'absorption des liquides corporelle et leur transfert rapide.

L'invention vise à réaliser de telles structures de transfert et des articles, en particulier des pansements, qui répondent aux objectifs énoncés ci-dessus.

L'invention cherche également à perfectionner des articles d'hygiène autres que des pansements, tels que des couches-culottes ou articles d'hygiène féminine, en offrant une vitesse d'absorption des fuites corporelles élevée, sans nuire au confort de l'utilisateur.

L'invention a pour objet, selon un premier de ses aspects, un article selon la revendication 1.

L'augmentation de la section transversale de la structure de transfert est obtenue de préférence par une augmentation de sa largeur, mieux par une augmentation continue, notamment linéaire de sa largeur sur une partie au moins dudit chemin. Le rapport (lₘₐₓ/lₘᵢₙ) de la largeur maximale (lₘₐₓ) à la largeur minimale (lₘᵢₙ) de la structure de transfert est de préférence supérieur ou égal à 1.5, de préférence 5, voire 10.

La section croissante de la structure de transfert permet d'augmenter la vitesse de transfert du liquide vers la zone de stockage et/ou d'évaporation.

L'invention permet, lorsque l'article est un pansement, d'optimiser le transfert d'une partie des exsudats absorbés par un pansement vers sa périphérie, afin de décharger le pansement de son excédent d'exsudat.

La structure de transfert est disposée de préférence à la surface d'une zone de captation, telle qu'une couche absorbante opposée à la plaie.

La structure de transfert permet d'étaler le liquide pour favoriser son évaporation, et ainsi d'éviter le retour du liquide vers la zone de captation et la plaie. La structure de transfert permet également d'acheminer le liquide vers au moins une partie absorbante formant zone de stockage, où le liquide peut s'accumuler.

La structure de transfert peut comporter au moins deux éléments distincts de transport du liquide vers la zone de stockage et/ou d'évaporation. Dans un mode de réalisation de l'invention, ces éléments sont par exemple disposés avec un décalage angulaire autour d'un centre de l'article, étant de préférence équirépartis angulairement. Dans un autre mode de réalisation de l'invention, les éléments s'étendent selon des axes sensiblement parallèle les uns aux autres. Par « sensiblement parallèle », on envisage tous les cas de figure où l'angle entre les axes de deux éléments n'est pas suffisant pour que ces axes ou leurs prolongements se coupent au niveau de l'article. Les éléments forment alors de préférence deux groupes d'éléments s'interpénétrant, notamment de façon alternée, au-dessus de la zone de captation.

Le fait que la structure de transfert comporte plusieurs éléments permet notamment que les éléments drainent le liquide dans des directions différentes, par exemple vers au moins deux zones de stockage et/ou d'évaporation respectives différentes.

La structure de transfert peut alors conduire le liquide vers une ou plusieurs zones de stockage et/ou d'évaporation périphériques, ce qui permet de s'adapter à des plaies très exsudatives, tout en conservant un pansement mince et conformable, sans altérer outre mesure ses propriétés de stockage et/ou d'évaporation.

Toujours afin d'optimiser la conformabilité de l'article, la structure de transfert peut être avantageusement non plane, notamment au niveau d'une partie absorbante réceptrice qui définit la zone de captation.

Les éléments de la structure de transfert peuvent être réalisés d'une seule pièce par découpe d'un matériau en feuille, notamment un papier ou un non-tissé, comportant des fibres absorbantes ; les éléments sont alors réunis entre eux par exemple au niveau de la zone de captation et/ou au niveau de zone de stockage et/ou d'évaporation.

Afin d'augmenter la capacité d'absorption de l'article, les éléments de la structure de transfert peuvent être au moins partiellement superposés, avec de préférence une barrière imperméable au liquide entre deux éléments se superposant, dans la région de superposition. Cette barrière prévient le transfert d'exsudat inter-éléments. La barrière peut être formée d'une couche d'un matériau imperméable au liquide, par exemple un film imperméable au liquide mais perméable à la vapeur d'eau.

L'invention peut permettre aussi, si cela est recherché, de minimiser les risques de fuites liés à l'accumulation de liquide dans une zone particulière de l'article, ainsi que les risques, dans le cas d'un pansement adhésif, de décollement du pansement sous l'effet de la gravité.

Le rapport de surface entre la partie absorbante réceptrice éventuelle et la structure de transfert est par exemple compris entre 50% - 50% et 66% - 33%, mieux 75 % - 25 %.

Cela peut permettre de réduire le risque de retour de liquide vers la zone de captation.

En présence de zones de stockage et/ou d'évaporation périphériques, un contact limité entre la structure de transfert et une zone de stockage et/ou d'évaporation périphérique peut également limiter le retour de liquide vers la zone de captation.

L'article est de préférence conditionné à l'état stérile, notamment lorsqu'il s'agit d'un pansement.

L'article selon l'invention peut se présenter, dans un exemple de mise en œuvre de l'invention, sous la forme d'un pansement qui comprend :
- un support imperméable à l'eau et perméable à la vapeur d'eau,
- une partie absorbante réceptrice au contact de la plaie, qui définit la zone de captation, et
- une structure de transfert selon l'invention, qui présente une section croissante en éloignement de la partie absorbante réceptrice.

### Partie absorbante réceptrice et partie absorbante formant zone de stockage et/ou d'évaporation

La partie absorbante réceptrice est encore qualifiée de « couche absorbante » et la partie absorbante formant zone de stockage et/ou d'évaporation de « couche formant réservoir ». Le terme « couche » doit se comprendre comme englobant un agencement monocouche ou plusieurs sous-couches assemblées.

La partie absorbante réceptrice et la partie absorbante formant zone de stockage et/ou d'évaporation présentent chacune de préférence une capacité d'absorption d'eau supérieure ou égale à 500 g/m², mieux à 800g/m². Elles peuvent comporter ou être constituées de tout matériau apte à stocker les liquides, comme par exemple les matériaux utilisés dans le domaine de l'hygiène et des pansements.

La partie absorbante formant zone de stockage et/ou d'évaporation est définie par un matériau ayant une capacité d'absorption du liquide supérieure à celle de la structure de transfert. Il est en est de même de la partie absorbante réceptrice. Ainsi, la capacité d'absorption d'eau ; en g/m², de la structure de transfert est inférieure à celle de la partie absorbante réceptrice et à celle de la partie absorbante formant zone de stockage et/ou d'évaporation.

On peut citer, à titre d'exemple, les mousses absorbantes et de préférence les mousses de polyuréthane hydrophiles, tous les matériaux à base de polymère superabsorbant (SAP), comme par exemple les non tissés absorbants incorporant des particules de SAP couramment utilisés dans le domaine de l'hygiène, les textiles absorbants comme par exemple les non tissés à base de viscose, de rayonne ou de cellulose, tels que par exemple une ouate, ou des hydrogels.

On préfère, dans le cadre de la présente invention, utiliser comme partie absorbante réceptrice un matériau alvéolaire, par exemple une mousse de polyuréthane hydrophile, à l'image par exemple de celle commercialisée sous la dénomination MCF.03 par la société Advanced Medical Solution.

Pour la partie absorbante formant zone de stockage et/ou d'évaporation, on préfère l'utilisation d'un matériau comportant un polymère superabsorbant SAP.

On préfère l'utilisation de non tissés obtenus par la méthode de fabrication par voie sèche, connue sous le nom de voie aérodynamique ou « airlaid », qui contiennent des particules de SAP et en particulier entre 20 à 60 % en poids de SAP par rapport au poids total du non tissé. De tels non tissés sont par exemple commercialisés par la société EAM Corporation sous la référence Novathin®.

Selon un mode préféré de mise en œuvre de l'invention, on utilise pour réaliser la partie absorbante formant zone de stockage et/ou d'évaporation un non tissé à base de particules de polymères superabsorbants et de fibres de cellulose sans incorporation de matériaux thermoliants ou de latex, et qui est recouvert sur chacune de ses faces par un voile cellulosique.

Selon une variante, on emploie comme matériau à base de SAP un matériau constitué de deux voiles cellulosiques entre lesquels sont incorporées des particules de polymères superabsorbants, seules ou en association avec des liants.

Selon une autre variante, on utilise un matériau à base de fibres de SAP seules ou en association avec des fibres non absorbantes. De préférence, ce matériau se présente sous forme d'un non tissé.

De préférence, la partie absorbante réceptrice est formée d'une couche absorbante recouverte d'une couche d'interface au contact de la plaie, qui évite d'altérer le processus de cicatrisation de la plaie lors du retrait du pansement. Cette couche d'interface est de préférence recouverte d'un voile protecteur provisoire, qui est retiré avant usage.

On peut utiliser pour une telle couche des pansements connus sous le nom de « pansements interface », comme par exemple les produits commercialisés par les sociétés Laboratoires URGO et MOLNLYCKE HEALTH CARE respectivement sous les dénominations URGOTUL® et MEPITEL®.

On peut aussi utiliser des couches perforées de formulations hydrophobe ou hydrophile, mais non absorbantes ou peu absorbantes, à base de polymères. Ces formulations peuvent être adhérentes ou non adhérentes.

On préfère utiliser des formulations micro adhérentes ou non adhérentes. De telles formulations sont bien connues de l'homme de l'art et sont par exemple réalisées à base de gel(s) de silicone, d'adhésif(s) siliconé(s) sensibles à la pression ou de compositions contenant un élastomère séquencé du type poly (styrène -oléfine - styrène), un plastifiant tel une huile minérale et une faible quantité d'hydrocolloïde(s) pour créer un environnement humide favorisant le processus de cicatrisation sans rendre la composition absorbante pour éviter de boucher les trous. De telles formulations micro adhérentes sont par exemple utilisées dans les pansements commercialisés par la société Laboratoires URGO sous les dénominations URGOCLEAN® et URGOTUL ABSORB®.

Selon des variantes possibles, ces pansements interfaces et ces couches perforées de formulation hydrophobe ou hydrophile sont associés à des matériaux perméables, en particulier des non tissés non absorbants, qui sont incorporés entre la couche absorbante et les couches supplémentaires.

### Structure de transfert

La structure de transfert est encore appelée « couche de distribution ».

Son rôle n'est pas de stocker les liquides mais de permettre leur étalement.

La structure de transfert est moins absorbante d'une part que la partie absorbante réceptrice et d'autre part que la partie absorbante formant zone de stockage et/ou d'évaporation. La structure de transfert présente de préférence une valeur d'absorption maximale qui est inférieure à 500 g/m².

De préférence, son épaisseur est comprise entre 50 et 1000 µm, mieux entre 300 et 500 µm.

La plus grande dimension de la structure de transfert est par exemple supérieure ou égale à 15 cm voire 30 cm. Cette plus grande dimension correspond à la longueur de la bande de transfert.

De préférence, la structure de transfert comporte ou est constituée d'une couche d'un matériau hydrophile.

De préférence, pour réaliser la structure de transfert, des matériaux fibreux à base de fibres absorbantes permettant l'étalement des liquides par diffusion latérale, qui sont couramment employés dans le domaine de l'hygiène et des pansements.

A titre d'exemple de tels matériaux, on peut citer les matériaux à base de fibres absorbantes d'origine végétale, telle que la viscose, la cellulose ou ses dérivés. Ces matériaux peuvent se présenter sous forme de tricots, de tissés ou de non tissés, obtenus par voie sèche ou par voie humide, comme les papiers.

La structure de transfert peut comporter un papier ou un non tissé, obtenu par voie sèche, à base de fibres absorbantes.

Dans le cadre de la présente invention, on préfère les non tissés et les papiers. Parmi les non tissés, on préfère ceux à base de fibres absorbantes, telles que la viscose ou la cellulose, associées à des fibres non absorbantes, comme par exemple des fibres de polyester ou de polyoléfine. A titre d'exemple de tels non tissés, on peut citer les produits commercialisés respectivement par les sociétés Suominen Corp et Orsa sous les dénominations Fibrella® 2000 et Jettex® 1205 c ou un non tissé hydrophile, composé de 55% cellulose 45% polyester, de référence DR870, fournisseur Berkshire, et dénommé par la suite non tissé Berkshire. Parmi les papiers on peut citer notamment le papier dit « Whatman », commercialisé par la société Whatman sous la référence Benchote plus. La structure de transfert peut se présenter sous la forme d'une bande drainante, directement en contact avec une couche d'un matériau absorbant de la partie absorbante réceptrice, sans couche intermédiaire entre les deux. En variante, une couche intermédiaire peut s'interposer, par exemple une couche ajourée d'un matériau hydrophobe, de façon à réduire le risque de retour de liquide vers la partie absorbante réceptrice.

La structure de transfert peut présenter des éléments constitutifs qui sont plus rapprochés les uns des autres au niveau de la zone de captation. Ces éléments peuvent être dans un même plan ou s'adapter au relief de la zone du corps ou de la forme de la plaie, d'où provient le liquide physiologique à capter.

### Support

L'article peut comporter un support, qui peut définir une partie au moins de la surface extérieure de l'article.

Le support est de préférence imperméable à l'eau et aux micro-organismes pathogènes extérieurs, tout en assurant une perméabilité à la vapeur d'eau, de manière à éviter à la fois le contact de la plaie avec des liquides extérieurs et des bactéries et la macération de la plaie. Le support est alors qualifié de « imperméable et respirant ».

Le support est de préférence mince et souple, de manière à mieux épouser la forme du corps et suivre les mouvements de celui-ci sans risquer de se détacher. Le support est avantageusement conformable. Son épaisseur peut être comprise entre 100 et 600 µm, de préférence entre 250 et 500 µm.

Le support peut être constitué d'un seul matériau ou de l'assemblage de plusieurs matériaux.

Le support peut ainsi comporter ou être constitué d'un film continu et imperméable aux liquides et aux bactéries mais perméable à la vapeur d'eau. Parmi les films utilisables, on peut citer à titre d'exemple les films en polyétheruréthane, en polyétheramide, ou en polyétherester. L'épaisseur du film est par exemple comprise entre 5 et 200 microns, de préférence entre 10 et 75 microns, de préférence encore entre 10 et 50 microns.

Le film présente avantageusement un taux de transmission de vapeur d'eau (MVTR : Moisture Vapor Transmission Rate) supérieur à 3 000 g/m²/24 heures, de préférence supérieur ou égal à 7 000 g/m²/24 heures, de préférence encore supérieur ou égal à 10 000 g/m²/24 heures. Une technique de mesure du taux de transmission de vapeur d'eau en contact liquide est décrite dans la norme NF-EN 13726-2 (Chapitre 3.3).

De tels films sont couramment utilisés dans la réalisation de pansements et sont par exemple constitués de films de polyuréthane, tels les films commercialisés par la société Exopack Advanced Coating sous la désignation INSPIRE.

Le film peut être remplacé par un complexe mousse/film. Le film peut aussi être complexé avec un autre matériau qui sert alors d'armature pour rigidifier le support.

Une telle armature peut permettre de rigidifier le support, de manière à ce qu'il ne s'enroule pas sur lui-même après retrait d'un voile protecteur pelable éventuel.

L'armature peut être constituée de tout matériau ajouré, tel un film perforé, un filet thermoplastique, un textile comme par exemple un tissé, un tricot ou un non tissé, de préférence élastique, pour une meilleure tenue de l'article sur la peau.

Lorsqu'un film perforé est utilisé pour réaliser l'armature, celui-ci est par exemple en polyéthylène ou en polypropylène. Lorsqu'un textile tissé est utilisé, celui-ci est par exemple en polyéthylène téréphtalate ou en polyamide. Le grammage de l'armature est de préférence compris entre 10 et 500 g/m², par exemple entre 20 et 300 g/m². Des pansements avec de tels supports avec armature sont décrits dans la demande WO 2012/140377.

Le film ou le complexe servant de support peut être assemblé aux autres couches du pansement à l'aide d'un adhésif discontinu, de manière à ne pas affecter la perméabilité du film ou du complexe à la vapeur d'eau.

L'invention a encore pour objet l'utilisation de l'article selon l'invention pour absorber un liquide physiologique. Ainsi, dans des exemples de mise en œuvre de l'invention, celle-ci s'applique à la réalisation de couches-culottes et d'articles d'hygiène féminine.

L'invention pourra être mieux comprise à la lecture de la description détaillée qui va suivre, d'exemples de mise en œuvre non limitatifs de celle-ci, et à l'examen du dessin annexé, sur lequel :
- la figure 1 représente de façon schématique, en coupe, un exemple d'article réalisé conformément à l'invention,
- la figure 2 représente isolément, en vue de dessus, la structure de transfert de l'exemple de la figure 1,
- les figures 3A à 3D sont des vues analogues à la figure 2, de variantes de la structure de transfert,
- la figure 4 est une vue analogue à la figure 1 d'une variante de réalisation d'article,
- la figure 5 illustre l'interpénétration d'éléments d'une variante de réalisation de la structure de transfert,
- les figures 6 à 8 représentent, en vue de dessus, d'autres variantes de structures de transfert,
- la figure 9 est une vue schématique, en perspective, partielle, d'un autre exemple de réalisation de la structure de transfert,
   les figures 10A et 13 illustrent les méthodologies pour réaliser les essais comparatifs, et
- les figures 10B,11, 12 et 14 sont des graphes ou tableaux illustrant des résultats expérimentaux.

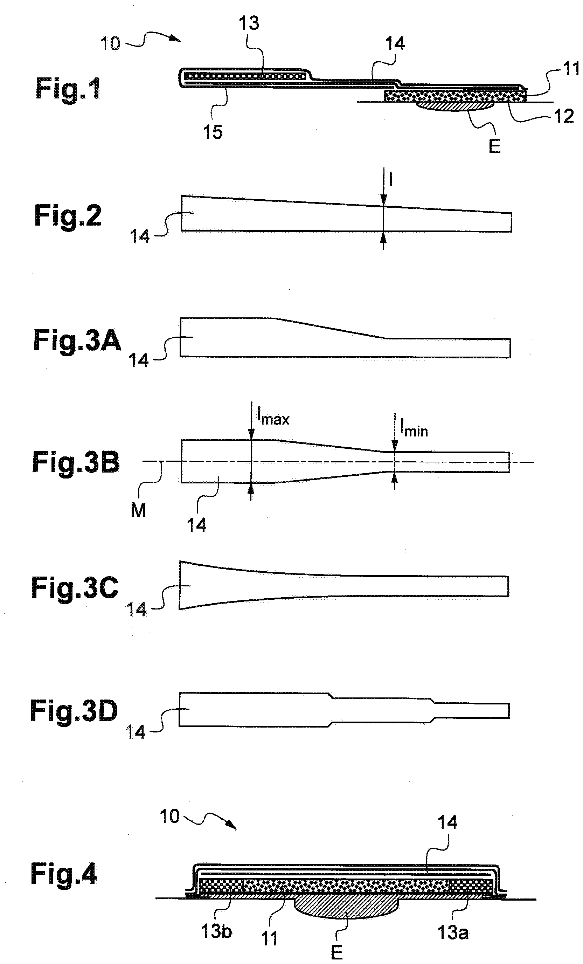

L'article 10 représenté à la figure 1 est destiné à être appliqué sur une partie du corps humain ou animal, pour absorber un liquide physiologique tel qu'un exsudat E. Il s'agit de préférence d'un pansement, destiné à être appliqué sur une plaie.

L'article 10 comporte, comme dans l'exemple illustré, une partie absorbante réceptrice 11, destinée à recevoir l'exsudat, définissant une zone de captation 12 de l'exsudat, et une partie absorbante formant zone de stockage et/ou d'évaporation 13, reliée à la partie absorbante réceptrice 11 par une structure de transfert 14 selon l'invention. Afin de réduire le risque de retour d'exsudat vers la partie absorbante réceptrice 11, l'article 10 peut comporter une couche intermédiaire telle que définie précédemment, non représentée sur le dessin, située entre la structure de transfert 14 et la partie absorbante réceptrice 11.

L'article 10 peut également comporter un support 15 constitué d'une couche imperméable à l'eau, s'étendant sous la structure de transfert 14, et pouvant également, comme illustré, recouvrir supérieurement la partie 13 formant zone de stockage et/ou évaporation et la structure de transfert 14.

La partie absorbante réceptrice 11, la structure de transfert 14 et le support 15 sont tels que définis plus haut.

La structure de transfert 14 présente des propriétés drainantes, permettant un transfert par diffusion du liquide depuis la partie absorbante réceptrice 11 vers la partie absorbante formant zone de stockage et/ou d'évaporation 13.

Conformément à un aspect de l'invention, la structure de transfert 14 comporte des fibres absorbantes et offre au transport de l'exsudat une section transversale croissante en direction de la partie formant zone de stockage et/ou évaporation 13.

Plus particulièrement, la structure de transfert 14 peut présenter en vue de dessus une forme générale trapézoïdale telle qu'illustrée à la figure 2, dont la largeur *l* croît de façon linéaire lorsque l'on se rapproche de la partie absorbante formant zone de stockage et/ou d'évaporation 13. Cette augmentation de la largeur *l* permet d'accroître la vitesse de diffusion du liquide, donc la quantité de liquide collectée par la partie absorbante formant zone de stockage et/ou d'évaporation 13, ce qui permet d'améliorer la performance de l'article dans la captation d'exsudat.

La structure de transfert 14 peut présenter une épaisseur sensiblement constante sur toute sa longueur.

L'invention n'est pas limitée à une variation linéaire de la largeur *l* d'une extrémité à l'autre de la structure de transfert 14 et cette dernière peut, comme illustré aux figures 3A à 3D, présenter par exemple une largeur *l* constante sur les portions de la structure de transfert 14 se superposant respectivement à la partie absorbante réceptrice 11 et à la partie absorbante formant zone de stockage et/ou d'évaporation 13, comme illustré à la figure 3A.

La variation de la largeur *l* peut être symétrique par rapport à une ligne médiane M, comme illustré à la figure 3B. La structure de transfert 14 peut présenter une forme générale symétrique par rapport à ladite ligne médiane M.

L'augmentation de la largeur *l* peut encore s'effectuer selon une fonction plus complexe, par exemple de profil non linéaire tel qu'un profil hyperbolique, parabolique ou autre, comme illustré à la figure 3C, voire par paliers, comme illustré à la figure 3D, avec une largeur constante sur chacun des paliers.

La variation de largeur peut s'effectuer entre des valeurs *lₘᵢₙ* et *lₘₐₓ*, avec par exemple un rapport *lₘₐₓ*/*lₘᵢₙ* supérieur ou égal à 50

L'épaisseur de la structure de transfert 14 peut varier, notamment augmenter en direction de la partie absorbante formant zone de stockage et/ou d'évaporation 13. Toutefois, l'épaisseur est de préférence constante, ce qui facilite la fabrication de l'article.

L'assemblage entre la partie absorbante réceptrice 11 et la structure de transfert 14 peut s'effectuer à l'aide d'un adhésif qui est par exemple appliqué de façon discontinue, Il en est de même de l'assemblage entre la structure de transfert 14 et la partie absorbante formant zone de stockage et/ou d'évaporation 13.

Le support 15 peut s'étendre sur une faible distance entre la partie absorbante réceptrice 11 et la structure de transfert 14. La section de recouvrement est par exemple inférieure à la surface, en vue de dessus, occupée par la partie absorbante réceptrice, d'un facteur inférieur à ½, voire inférieur à ¼.

L'article 10 peut ne comporter, comme illustré aux figures 1, 4, 6 et 7, 9, qu'une seule partie formant zone de stockage et/ou évaporation 13, qui est déportée par rapport à la partie absorbante réceptrice 11, de sorte qu'en vue de dessus il n'existe aucune superposition entre la partie formant zone de stockage et/ou évaporation 13 et la partie absorbante réceptrice 11. Dans ces modes de réalisation, la zone de stockage et/ou d'évaporation 13 est disposée en bordure ou à la périphérie de la zone absorbante réceptrice 11, sans superposition entre ces deux zones.

Dans la variante illustrée à la figure 4, l'article 10 comporte deux parties absorbantes opposées formant zone de stockage et/ou d'évaporation 13a et 13b, qui sont disposées respectivement de part et d'autre de la partie absorbante réceptrice 11.

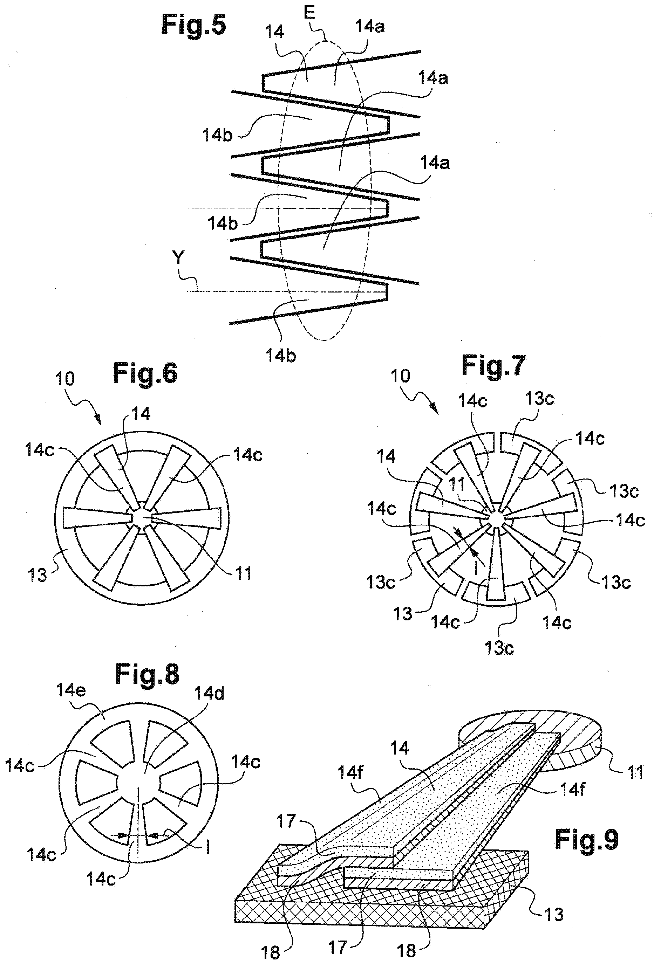

La structure de transfert 14 peut comporter plusieurs éléments de transfert 14a, 14b qui s'interpénètrent au-dessus de la partie absorbante réceptrice 11, comme illustré à la figure 5. Le fait que chaque élément 14a ou 14b présente une largeur croissante facilite l'imbrication des éléments et donne la possibilité d'avoir des éléments de transfert rapprochés, bien que disjoints, au niveau de la partie absorbante réceptrice 11.

Les éléments de transfert 14a sont par exemple d'axes longitudinaux Y parallèles entre eux, de même que les éléments 14b.

Les éléments 14a, 14b sont par exemple découpés d'une seule pièce dans une feuille d'un papier ou non tissé comportant des fibres absorbantes comme détaillé plus haut. Les éléments 14a peuvent se raccorder entre eux à une extrémité, qui est superposée à la partie absorbante formant zone de stockage et/ou d'évaporation 13a. Il en est de même des éléments 14b.

Les parties absorbantes formant zones de stockage et/ou d'évaporation 13a et 13b peuvent être disjointes. En variante, celles-ci se rejoignent, et entourent par exemple complètement la partie absorbante réceptrice 11.

Le support 15 peut recouvrir extérieurement les parties absorbantes formant zones de stockage et/ou d'évaporation 13a, 13b et la partie absorbante réceptrice 11, sauf sur la face de celle-ci en contact avec la plaie.

On a représenté à la figure 6 une variante de réalisation dans laquelle la structure de transfert 14 comporte des éléments de transfert radiaux 14c, qui présentent chacun une largeur *l* augmentant depuis la zone de captation 11, qui est centrale, vers la partie absorbante formant zone de stockage et/ou d'évaporation 13, qui est périphérique et de forme continue annulaire autour de la zone de captation 11.

Les éléments 14c sont par exemple disposés de façon équiangulairement répartie autour de la zone de captation 11.

La partie formant zone de stockage et/ou évaporation 13 peut être de forme annulaire continue angulairement, comme illustré à la figure 6 ou discontinue angulairement, comme illustré à la figure 7, chaque élément 14c de la structure de transfert 14 pouvant alors communiquer avec un unique élément respectif 13c formant réservoir.

Dans la variante illustrée à la figure 8, les éléments 14c de la structure de transfert 14 sont réalisés d'une seule pièce avec une partie centrale 14d qui se superpose à la zone de captation et avec une partie périphérique 14e qui est reliée à la partie formant zone de stockage et/ou évaporation et/ou définit une zone d'évaporation.

La largeur *l* de chaque élément 14c, mesurée perpendiculairement au rayon correspondant, augmente continûment depuis la partie centrale 14d vers la partie périphérique 14e.

Dans les exemples qui viennent d'être décrits, comportant une structure de transfert 14 comportant plusieurs éléments reliant la zone de captation à une ou plusieurs parties formant zone de stockage et/ou évaporation, ces éléments sont disjoints et non superposés.

On a illustré à la figure 9 la possibilité pour la structure de transfert 14 de comporter deux éléments 14f qui se superposent au moins partiellement. Chaque élément 14f comporte une couche drainante 18 qui est recouverte supérieurement d'une couche barrière 17, imperméable à l'eau, de telle sorte que le liquide qui diffuse dans la couche drainante 18 ne puisse pas gagner l'élément de transfert qui le recouvre.

Une superposition des éléments drainants peut permettre d'accroître le débit du liquide qui diffuse dans la structure de transfert, pour un même encombrement sur la peau.

### Essais comparatifs

Pour mettre en évidence le fait que la forme de la structure de transfert selon l'invention permet d'augmenter la vitesse de drainage d'un liquide d'un point d'entrée vers un point de sortie, des essais comparatifs ont été réalisés, qui établissent qu'une bande de forme triangulaire initialement sèche se mouille plus rapidement qu'une bande de forme rectangulaire de même surface. Il en est de même lorsque la bande est initialement mouillée. Le liquide est introduit à l'extrémité la plus étroite pour la bande de forme triangulaire.

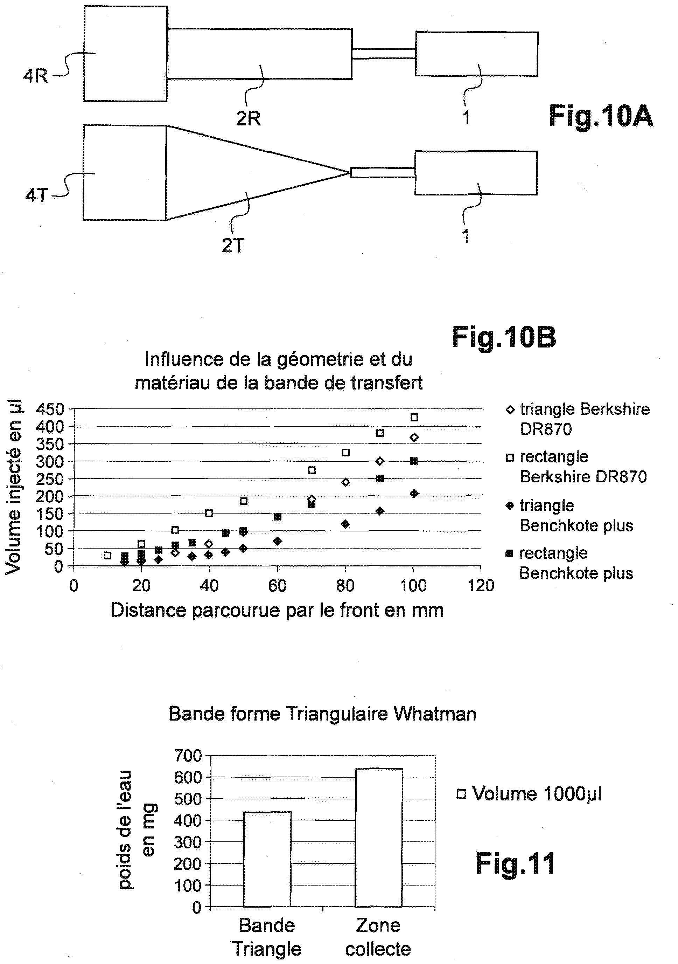

La méthodologie pour l'établir a été la suivante. Comme décrit sur la figure 10A, le liquide est délivré à la bande à tester (2R, 2T) par un pousse seringue 1, jusqu'à une partie formant zone de stockage et/ou évaporation (4R, 4T). Dans cet essai, la zone de stockage (ou réservoir) est réalisée dans le même matériau que la bande à laquelle il est associé.

Après avoir délivré un volume prédéfini avec le pousse seringue, on mesure le liquide dans la bande de transfert (2R, 2T) et dans la partie formant réservoir (4R, 4T) par pesée.

On a pu comparer les quantités de liquide présentes dans des bandes de forme triangulaire et rectangulaire, ainsi que dans les parties formant zones de stockage et/ou évaporation.

Le débit d'injection s'élève à 10 µL par minute. La bande testée est soit rectangulaire (dimensions 45 mm ^{∗} 210 mm) soit triangulaire (base 90 mm - hauteur 210 mm). On note que les deux bandes testées ont la même épaisseur (épaisseur d'environ 100 µm), et donc le même volume. On détermine:
- l'avancement du front du liquide au sein de la bande de transfert en fonction du volume injecté, et cela avant que le liquide n'atteigne le réservoir
- après que le liquide a atteint le réservoir, la masse du réservoir, cette dernière étant comparée à la masse totale injectée.

La figure 10B représente la distance parcourue par le front de liquide dans la bande de transfert, et cela pour deux matériaux différents : non tissé Berkshire (fournisseur Berkshire - référence BR870) et Papier Benchkote plus (fournisseur Whatman). Ces deux matériaux sont des matériaux comportant des fibres absorbantes. On peut constater, à l'examen de la figure 10, que pour une même quantité d'eau injectée, la distance parcourue par le front de liquide (en millimètres) est supérieure dans le cas de la bande de forme triangulaire 2T, et cela pour les deux matériaux testés. Cela signifie que le liquide migre plus vite dans la bande de forme triangulaire 2T, en particulier lorsque le volume injecté dépasse 40 µl.

Cela permet au liquide de gagner plus rapidement la partie formant zone de stockage et/ou évaporation 4T. Le pouvoir drainant de la bande de transfert triangulaire 2T est supérieur au pouvoir drainant de la bande de transfert rectangulaire 2R.

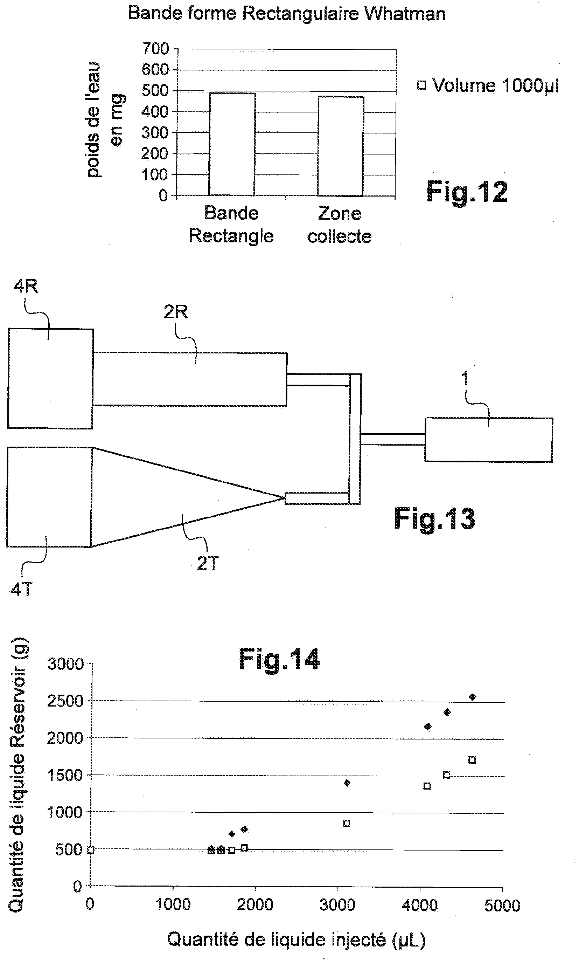

On a représenté, sur la figure 11, les volumes de liquide accumulés après injection d'un volume de liquide de 1 ml, dans le cas où la bande de transfert et le réservoir sont en non tissé Berkshire. Ces volumes ont été estimés en découpant la bande de transfert et le réservoir de collecte et en les pesant. La figure 12 présente des résultats similaires, obtenus en mettant en œuvre la bande de transfert rectangulaire en papier Whatman. On constate que le réservoir de collecte contient plus de liquide lorsque la bande de transfert triangulaire a été mise en œuvre. Ainsi, lorsque la bande présente une forme triangulaire, le volume de liquide collecté dans le réservoir, à un instant donné, est supérieur à celui collecté dans le réservoir raccordé à une bande rectangulaire.

Un autre essai a été réalisé, en utilisant un montage proche de celui des essais précédemment décrits, et représenté sur la figure 13. Une bande de transfert 2R, 2T est raccordée, à l'une de ses extrémités, à un dispositif d'injection de liquide 1, et, à l'extrémité opposée, à un réservoir de stockage 4R, 4T, constitué d'un matériau Airlaid, précédemment décrit, commercialisé par la société Buckeye Steinfurt GmbH sous la référence Vizorb® 3924. Les bandes de transfert ont des dimensions identiques à celles décrites dans l'essai précédent. Elles sont constituées de non tissé Berkshire. Pour différentes quantités de liquide injectées, on pèse le réservoir de stockage, afin de déterminer la quantité de liquide drainée par la bande de transfert.

La figure 14 représente la quantité de liquide dans le réservoir, exprimée en grammes, en fonction de la quantité de liquide injectée dans la bande de transfert, exprimée en µl, et cela pour une bande de transfert triangulaire et pour une bande de transfert rectangulaire. Les bandes de transfert ont des dimensions analogues à celles décrites dans l'exemple précédent. Leurs surfaces et leurs épaisseurs sont identiques, si bien que les bandes de transfert ont le même volume.

La masse initiale du réservoir 4R, 4T est de 500 g. Au-delà d'un certain volume de liquide injecté, le liquide atteint le réservoir, la masse de ce dernier augmentant. Dès lors, on constate que le liquide atteint plus rapidement le réservoir lorsqu'on utilise une bande triangulaire 2T. Cela montre que la bande triangulaire 2T a un effet drainant supérieur à la bande rectangulaire 2R.

L'invention peut s'appliquer à des articles autres que destinés à être appliqués sur une plaie, par exemple des couches-culottes ou des articles d'hygiène féminine.

## Revendications

1. Article (10) ayant des propriétés d'absorption d'un liquide physiologique, comportant une zone de captation du liquide et une structure de transfert (14), cette dernière comportant des fibres absorbantes et étant agencée pour transporter le liquide de ladite zone de captation (12) vers au moins une zone de stockage et/ou d'évaporation (13), ladite structure de transfert (14) présentant une section transversale croissante en direction de ladite zone de stockage et/ou d'évaporation, sur une partie au moins du chemin séparant ladite zone de captation de ladite zone de stockage et/ou d'évaporation et ayant une capacité d'absorption du liquide inférieure d'une part à une partie absorbante formant la zone de stockage et/ou d'évaporation et d'autre part à une partie absorbante réceptrice définissant la zone de captation du liquide.

2. Article selon la revendication 1, l'augmentation de la section étant obtenue par une augmentation de la largeur (/) de la section, de préférence la largeur (/) augmentant de façon continue, notamment linéaire sur une partie au moins dudit chemin.

3. Article selon l'une quelconque des revendications précédentes, le rapport (lₘₐₓ/lₘᵢₙ) de la largeur maximale (lₘₐₓ) à la largeur minimale (lₘᵢₙ) étant supérieur ou égal à 1.5, de préférence 5 voire 10.

4. Article selon l'une quelconque des revendications précédentes, l'épaisseur de la structure de transfert (14) étant comprise entre 50 et 1000µm.

5. Article selon l'une quelconque des revendications précédentes, la structure de transfert présentant une valeur d'absorption maximale inférieure à 500 g/m².

6. Article selon l'une quelconque des revendications précédentes, la structure de transfert (14) comportant au moins deux éléments distincts (14a ; 14b ; 14c) de transport du liquide vers la zone (13) de stockage et/ou d'évaporation.

7. Article selon la revendication 6, lesdits éléments étant disposés avec un décalage angulaire autour d'un centre de l'article, notamment étant équirépartis angulairement.

8. Article selon la revendication 6, lesdits éléments s'étendant selon des axes sensiblement parallèles les uns aux autres.

9. Article selon l'une quelconque des revendications précédentes, la structure de transfert (14) comportant des éléments drainant le liquide dans des directions différentes, notamment vers au moins deux parties (13a, 13b) formant zones de stockage et/ou d'évaporation respectives différentes.

10. Article selon la revendication 9, lesdits éléments s'interpénétrant au niveau de la zone de captation.

11. Article selon l'une des revendications 9 et 10, les éléments étant réalisés d'une seule pièce par découpe d'un matériau en feuille, notamment un papier ou non-tissé.

12. Article selon la revendication 11, les éléments étant réunis entre eux au niveau de la zone de captation.

13. Article selon la revendication 11 ou 12, les éléments étant réunis entre eux au niveau de la zone de stockage et/ou d'évaporation.

14. Article selon l'une quelconque des revendications précédentes, la partie absorbante réceptrice comprenant un matériau alvéolaire.

15. Article selon l'une quelconque des revendications précédentes, la structure de transfert comportant un non tissé, obtenu par voie sèche, à base de fibres absorbantes ou un papier.

## Patentansprüche

1. Artikel (10) mit Eigenschaften zur Absorption einer physiologischen Flüssigkeit, umfassend eine Flüssigkeitsaufnahmezone und eine Transferstruktur (14), wobei Letztere absorbierende Fasern umfasst und dazu vorgesehen ist, die Flüssigkeit von der Aufnahmezone (12) zu mindestens einer Speicher- und/oder Verdampfungszone (13) zu transportieren, wobei die Transferstruktur (14) auf mindestens einem Teil der die Aufnahmezone von der Speicher- und/oder Verdampfungszone trennenden Strecke einen in Richtung der Speicher- und/oder Verdampfungzone zunehmenden Querschnitt aufweist und eine Kapazität zur Aufnahme der Flüssigkeit hat, die geringer als die eines die Speicher- und/oder Verdampfungszone bildenden absorbierenden Teils einerseits und die eines aufnehmenden absorbierenden Teils, der die Zone zu Aufnahme der Flüssigkeit definiert, andererseits ist.

2. Artikel nach Anspruch 1, wobei die Querschnittsvergrößerung durch eine Vergrößerung der Breite (/) des Querschnitts erhalten wird, vorzugsweise der Breite (/), die kontinuierlich, insbesondere linear auf mindestens einem Teil der Strecke zunimmt.

3. Artikel nach einem der vorhergehenden Ansprüche, wobei das Verhältnis (lₘₐₓ/lₘᵢₙ) der maximalen Breite (lₘₐₓ) zur minimalen Breite (lₘᵢₙ) größer oder gleich 1,5, vorzugsweise 5 oder sogar 10 beträgt.

4. Artikel nach einem der vorhergehenden Ansprüche, wobei die Dicke der Transferstruktur (14) zwischen 50 und 1000 µm liegt.

5. Artikel nach einem der vorhergehenden Ansprüche, wobei die Transferstruktur einen maximalen Absorptionswert von weniger als 500 g/m² aufweist.

6. Artikel nach einem der vorhergehenden Ansprüche, wobei die Transferstruktur (14) mindestens zwei verschiedene Elemente (14a; 14b; 14c) zum Transportieren der Flüssigkeit zu der Speicher- und/oder Verdampfungszone (13) umfasst.

7. Artikel nach Auspruch 6, wobei die Elemente mit einem Winkelversatz um ein Mitte des Artikels herum angeordnet und insbesondere gleichmäßig winkelförmig verteilt sind.

8. Artikel nach Anspruch 6, wobei die Elemente entlang im Wesentlichen zueinander paralleler Achsen angeordnet sind.

9. Artikel nach einem der vorhergehenden Ansprüche, wobei die Transferstruktur (14) Elemente umfasst, die die Flüssigkeit in verschiedene Richtungen ableiten, insbesondere zu mindestens zwei Teilen (13a, 13b), die jeweils unterschiedliche Speicher- und/oder Verdampfungszonen bilden.

10. Artikel nach Anspruch 9, wobei die Elemente im Bereich der Aufnahmezone in Wechselwirkung stehen.

11. Artikel nach einem der Ansprüche 9 und 10, wobei die Elemente durch Schneiden eines Blattmaterials, insbesondere eines Papiers oder Vliesstoffs, aus einem einzigen Stück hergestellt sind.

12. Artikel nach Anspruch 11, wobei die Elemente im Bereich der Aufnahmezone zusammengefügt sind.

13. Artikel nach Anspruch 11 oder 12, wobei die Elemente im Bereich der Speicher- und/oder Verdampfungszone zusammengefügt sind.

14. Artikel nach einem der vorstehenden Ansprüche, wobei der aufnehmende absorbierende Teil ein Wabenmaterial umfasst.

15. Artikel nach einem der vorhergehenden Ansprüche, wobei die Transferstruktur einen Trockenvliesstoff aus absorbierenden Fasern oder Papier umfasst.

## Claims

1. Article (10) having properties of absorption of a physiological liquid, having a liquid-collecting zone and a transfer structure (14), the latter having absorbent fibres and being designed to transport the liquid from said collecting zone (12) to at least one storage and/or evaporation zone (13), said transfer structure (14) having a cross section increasing in the direction of said storage and/or evaporation zone, over at least part of the path separating said collecting zone from said storage and/or evaporation zone, and having a liquid absorption capacity that is less, on the one hand, than an absorbent part forming the storage and/or evaporation zone and, on the other hand, than a receiving absorbent part defining the liquid-collecting zone.

2. Article according to Claim 1, the increase in the cross section being obtained by an increase in the width (*l*) of the cross section, the width (*l*) preferably increasing continuously, in particular linearly on at least part of said path.

3. Article according to either of the preceding claims, the ratio (lₘₐₓ/lₘᵢₙ) of the maximum width (lₘₐₓ) to the minimum width (lₘᵢₙ) being greater than or equal to 1.5, preferably 5, or even 10.

4. Article according to any one of the preceding claims, the thickness of the transfer structure (14) being between 50 and 1000 µm.

5. Article according to any one of the preceding claims, the transfer structure having a maximum absorption value of less than 500 g/m².

6. Article according to any one of the preceding claims, the transfer structure (14) having at least two separate elements (14a; 14b; 14c) for transporting the liquid to the storage and/or evaporation zone (13).

7. Article according to Claim 6, said elements being arranged with an angular offset about a centre of the article, in particular being distributed at uniform angles.

8. Article according to Claim 6, said elements extending along axes substantially parallel to each other.

9. Article according to any one of the preceding claims, the transfer structure (14) having elements draining the liquid in different directions, in particular to at least two parts (13a, 13b) that form respective different storage and/or evaporation zones.

10. Article according to Claim 9, said elements interpenetrating one another at the collecting zone.

11. Article according to either of Claims 9 and 10, the elements being produced in one piece by cutting from a material in sheet form, in particular a paper or nonwoven.

12. Article according to Claim 11, the elements being joined together at the collecting zone.

13. Article according to Claim 11 or 12, the elements being joined together at the storage and/or evaporation zone.

14. Article according to any one of the preceding claims, the receiving absorbent part comprising a cellular material.

15. Article according to any one of the preceding claims, the transfer structure having a nonwoven, obtained by a dry process, based on absorbent fibres or a paper.
